(19) Europäisches Patentamt

European Patent Office

Office européen des brevets

(11) Veröffentlichungsnummer: **0 398 083**

**A1**

(12) **EUROPÄISCHE PATENTANMELDUNG**

(21) Anmeldenummer: **90108386.5**

(22) Anmeldetag: **03.05.90**

(51) Int. Cl.5: **C12M 1/12, C12M 3/00**

(30) Priorität: **06.05.89 DE 3914956**

(43) Veröffentlichungstag der Anmeldung:
**22.11.90 Patentblatt 90/47**

(84) Benannte Vertragsstaaten:
**DE FR GB IT**

(71) Anmelder: **Fischer, Karl-Heinz
Am Kochberg 14
D-6457 Maintal 3(DE)**

Anmelder: **Bauer, Hermann, Dr.
Brahmsweg 19**

**D-7400 Tübingen(DE)**

(72) Erfinder: **Fischer, Karl-Heinz
Am Kochberg 14
D-6457 Maintal 3(DE)**
Erfinder: **Bauer, Hermann, Dr.
Brahmsweg 19
D-7400 Tübingen(DE)**

(74) Vertreter: **Jochem, Bernd, Dipl.-Wirtsch.-Ing.
Patentanwälte Beyer & Jochem Postfach 17
01 45
D-6000 Frankfurt/Main(DE)**

(54) **Verfahren zur Beschleunigung des Stoffaustauchs eines kontinuierlichen Bioreaktors und Vorrichtung zur Durchführung dieses Verfahrens.**

(57) Es wird ein Verfahren zur Beschleunigung des Stoffaustauschs eines kontinuierlichen Bioreaktors vorgeschlagen, bei welchem innerhalb einer Flüssigkeit ein Reaktionsraum (26) durch eine semipermeable Membran (11) von einem Versorgungsraum (24) getrennt ist. Bisher wurde der Stoffaustausch durch die Membran (11) dadurch beeinträchtigt, daß sich während der Betriebszeit auf seiten des Reaktionsraums vor der Membranwand ein Konzentrationsgradient der durch die Membran zurückgehaltenen Stoffe ausbildete. Diese sog. Sekundärfilterschicht drosselte dann den Stoffaustausch. Um sie zu vermeiden, wird vorgeschlagen, daß die Bewegungsrichtung der die Membran (11) durchdringenden Stoffe durch Druckänderungen in zeitlichen Abständen vorübergehend umgekehrt wird. Diese Maßnahme führt zu einer Ablösung der Sekundärfilterschicht, ohne daß die sie bildenden Teilchen schädlichen Scherkräften ausgesetzt sind. Vorzugsweise finden Kapillarmembranen (11) Anwendung, deren Innenraum (24) den Versorgungsraum bildet.

Fig. 7

**EP 0 398 083 A1**

## Verfahren zur Beschleunigung des Stoffaustauschs eines kontinuierlichen Bioreaktors und Vorrichtung zur Durchführung dieses Verfahrens

Bioreaktoren finden Anwendung in der Biotechnologie für eine Vielzahl biologischer Umsetzungen. So werden z. B. biochemische Umsetzungen mit Hilfe von Biokatalysatoren in Bioreaktoren als Einzel- oder Mehrfachreaktionen, beispielsweise enzymatische Hydrolysen u. dgl. durchgeführt. Bei der Biosynthese von Naturstoffen, wie z. B. Peptidhormonen, Antibiotika usw., enthalten die Bioreaktoren die zur Produktion dieser Stoffe eingesetzten Zellkulturen und in ähnlicher Weise bei zellfreien Biosynthesen z. B. Zellextrakte mit und ohne Zusatz definierter Synthetasen.

Im Sinne der vorliegenden Anmeldung werden auch solche Reaktions- bzw. Brutgefäße als Bioreaktoren bezeichnet, die auf den Gebieten der Mikrobiologie, Histologie, Bakteriologie, Virologie usw. sowie der Gentechnologie zum Anlegen von Zellkulturen unter steriler Ver- und Entsorgung ohne Zellkulturverluste, zur Aufzucht, Ver- und Entsorgung bestimmter - auch riskioreicher - Stämme und einer damit betriebenen Produktion bestimmter Stoffe dienen.

Innerhalb des vorstehend umrissenen Anwendungsbereichs betrifft die Erfindung ein Verfahren zur Beschleunigung des Stoffaustauschs eines kontinuierlichen Bioreaktors, bei welchem innerhalb einer Flüssigkeit ein Reaktionsraum durch eine semipermeable Membran von einem Versorgungsraum getrennt ist.

Damit unterscheidet sich das erfindungsgemäße Verfahren von vornherein von diskontinuierlich betriebenen Verfahren. Diese verwenden in einfachster Form einen Behälter, z. B. ein Glas oder eine Flasche, in dem eine biologische Umsetzung, beispielsweise das Vergären pflanzlicher Produkte zur Gewinnung von Alkohol (Wein, Bier, usw.) stattfindet. Bei diskontinuierlichen Verfahren wird der Umsetzungsprozeß gestartet, und erst nachdem die Reaktion zum Stillstand gekommen ist, wird das gewünschte Produkt aufgearbeitet, z. B. destilliert, abgepreßt usw. Während der Reaktion werden keine Produkte entnommen, und nach der Reaktion wird das produzierende System meist verworfen. Die Reaktionsgleichgewichte liegen häufig so ungünstig, daß die Reaktion vorzeitig zum Stillstand kommt und dadurch ein vollständiger Umsatz nicht erreicht wird oder die Ausbeute insgesamt weit hinter der theoretisch möglichen zurückbleibt.

Um die gravierenden Nachteile der diskontinuierlichen Verfahren zu vermeiden, sind kontinuierliche Bioreaktoren entwickelt worden. Hierbei werden dem Reaktor kontinuierlich bestimmte Ausgangsstoffe zugeführt, d. h. der Reaktor wird "gefüttert", und die umgesetzten Produkte werden in gleichem Maße abgeführt. Hierzu müssen die "produzierenden Systeme" immobilisiert oder separiert werden, da sonst mit dem Abführen ein Verlust des produzierenden Systems einhergehen würde. Als produzierendes System kann prinzipiell alles eingesetzt werden, was in der Lage ist, die Umsetzung bzw. Synthese gewünschter Verbindungen zu ermöglichen oder zu beschleunigen. Im allgemeinen sind dies Enzyme und Enzym-Komplexe sowie ganze Zellen und Zellverbände oder auch nur ein Zellextrakt ohne genau definierte Zusammensetzung.

Die Immobilisierung solcher produzierender Systeme kann z. B. durch Einschließen in unlösliche Matrices, die jedoch für die zu- und abzuführenden Stoffe durchlässig sind, erfolgen. Des weiteren wird auch die Immobilsierung durch Adsorption und chemische Bindung an sich neutral verhaltende Träger, z. B. Schüttmaterialien, häufig eingesetzt. Die Erfindung geht von einer Immobilisierung des produzierenden Systems durch Kompartimentierung mittels einer semiper meablen Membran aus, wie dies bei der Stofftrennung durch die sog. Membranfiltration der Fall ist.

Als Membranen werden heute eine Vielzahl von Werkstoffen und Strukturen bezeichnet, vom Filter bis zu Druckmembranen. Im Zusammenhang mit der Erfindung geht es jedoch nur um Membranen, die dem Durchtritt verschiedener Stoffe unterschiedlichen Widerstand entgegensetzen, d. h. der Stofftrennung dienen. Somit ist eine klare Trennung zu undurchlässigen Membranen, z. B. zur Druckübertragung gezogen. Unscharf dagegen ist die Unterscheidung von Membranen zu Filtern und Feinstfiltern, d. h. es kommt in Grenzbereichen zu Überschneidungen. Von der Stoffart, Durchlässigkeit und Technik können Feinstfilter und Membranen im engeren Sinne dieselben Eigenschaften und Funktionen haben. Werden die Poren in solchen Filtern kleiner als 1 μm, so werden rasch Rückhaltevermögen in der Größenordnung von Molekülen erreicht, und man spricht von Ultra-(Membran)-Filtration. Dadurch ist es möglich, größere von kleineren Molekülen abzutrennen oder Kompartimente zu schaffen, in denen größere Moleküle "immobilisiert" werden, während kleinere lediglich die Barriere der Membran bzw. des Feinstfilters durch Diffusion oder Druckgefälle (Fluß) überwinden müssen. Der ( auf diese Funktion abzielende Begriff der semipermeablen Membran soll hier deshalb auch entsprechend wirkende Feinstfilter umfassen.

Wird mit einem Druckgefälle gearbeitet, kommt es an der Grenzfläche über der Membran zur Ausbildung eines Konzentrationsgradienten der Moleküle oder sonstigen verhältnismäßig großen Partikel des produzierenden Systems, da diese nicht durch die Membran hindurchtreten können. Hierdurch wird ein osmotischer Gegendruck aufgebaut, der den erforderlichen Betriebsdruck weiter erhöht.

Gelingt es nicht, diesen Gradienten durch starkes Vermischen direkt an der Grenzfläche zu unterdrükken, so muß der Fluß entsprechend reduziert werden. Andernfalls wird das Löslichkeitsprodukte der Makromoleküle rasch überschritten, und es bildet sich eine Deckschicht aus ungelösten Bestandteilen, die sog. Sekundärfilterschicht.

Geht man davon aus, daß der Rücktransport der ungelösten, suspendierten oder gelösten Partikel bzw. Makromoleküle aus der Deckschicht in den Kern der Rohlösung bzw. der Biomasse nicht nur auf reiner Diffusion beruhen sollte, da sich sonst bei Makromolekülen inakzeptabel große Austauschzeiten ergeben, so läßt sich z. B. durch Rühren der Rücktransport intensivieren. Dabei führen Scherkräfte, die von der parallel zur Membranoberfläche strömenden Lösung auf die gebildete Deckschicht ausgeübt werden, zu deren Erosion. Der weitere Rücktransport erfolgt dann sowohl durch Diffusion (gegen den Filtrationsstrom) in die Rohlösung als auch durch Konvektion z. B. infolge der Rührströmung.

Da vor allem im biochemischen Bereich der kontinuierliche Abbau einer sich bildenden Sekundärfilterschicht durch Rühren wegen der genannten Scherkräfte die Zerstörung der biologischen Aktivität der Biomakromoleküle (z. B. Enzyme etc.) zur Folge haben kann, müssen entweder die Nachteile ungerührter Systeme oder eine Beeinträchtigung der Lebensdauer der Biosysteme in Kauf genommen oder aber Systeme mit extrem großer Oberfläche eingesetzt werden, um bei geringerem Fluß arbeiten zu können. Alle diese Lösungen sind mehr oder weniger unbefriedigend.

In der Biotechnologie werden in Bioreaktoren mit Ultramembranfiltern Zellkulturen u. dgl. über solche Membranen "gefüttert" und "entsorgt". Nachteilig ist auch hier die Bildung einer Sekundärfilterschicht. Außerdem besteht die Problematik einer intensiven Durchmischung, ohne die produzierenden Systeme (z. B. Zellen) zu schädigen. Erfolgt der Austausch zwischen dem das zuführende/abführende System enthaltenden Versorgungsraum auf der einen und dem das produzierende System enthaltenden Reaktionsraum auf der anderen Seite der semipermeablen Membran nur über Diffusion, so können nur extrem geringe Austauschraten erzielt werden. Eine aktive Beeinflussung der Reaktionskinetik über das Fließsystem ist damit praktisch ausgeschlossen.

Großen Einfluß auf die Kapazität eines Bioreaktors haben auch die Struktur und äußere Form der Membran. Vorzugsweise verwendet man asymmetrisch aufgebaute Membranen, bei denen die eigentliche Filterschicht auf einem makroporösen Träger aufgezogen ist. Die Filtrationsrichtung ist damit festgelegt. Zuerst muß die Filterschicht durchströmt werden, da im umgekehrten Falle die größeren Poren des Trägers systematisch verstopft würden.

Planare Membranen besitzen eine relativ geringe Membranfläche im Verhältnis zum Mindestbedarf an Raum und Lösungsmittelmenge. Wesentlich günstigere Verhältnisse ergeben sich bei den sog. Kapillarmembaren. Bisher ist hierbei die eigentliche Filtrationsschicht stets auf der Innenoberfläche aufgezogen. Werden solche Kapillarmembranfilter durchströmt betrieben, so verringert sich zwar die Sekundärfilterschicht, denn der Durchfluß begünstigt den radialen Stoffaustausch und führt zu einer besseren Durchmischung, da turbulente bzw. quasi-turbulente Flußprofile entstehen. Biologische Systeme, d. h. Biomakromoleküle und Zellen, sind jedoch den unter diesen Bedingungen wirkenden Scherkräften nicht gewachsen und verlieren ihre biologische Aktivität und damit ihre Wirkungsweise. Soll sie erhalten bleiben, muß bei Kapillarmembranen mit so langsamer Durchströmung gearbeitet werden, daß zumindest im direkten Grenzbereich der Stoffaustausch in der Sekundärfilterschicht vor wiegend nur über Diffusionsmechanismen abläuft.

Werden die bekannten Kapillarmembranen mit radial innen angeordneter Filtrationsschicht für Bioreaktoren eingesetzt, so muß sich aus den vorstehend genannten Gründen das produzierende System im Inneren der Kapillaren befinden. Die Zuführung der Ausgangsstoffe radial von außen ins Innere der Kapillaren ist dabei unproblematisch. Bei der Abfuhr der produzierten Stoffe mittels Diafiltration radial von innen nach außen durch die Kapillarwände hindurch kommt es jedoch, weil Scherkräfte vermieden werden müssen, zur Ausbildung der bereits angesprochenen Sekundärfilterschicht, und der Umsatz sinkt rasch ab. Im Extremfall kann es sogar zum totalen Erliegen der Reaktion kommen.

Derartige Bioreaktoren mit den bekannten Kapillarmembranen mit innenliegender Filtrationsschicht haben darüber hinaus den weiteren Nachteil, daß die Phasen- bzw. Volumenverhältnisse zwischen dem produzierenden System im Inneren der Kapillarmembranen und dem zu-/abführenden System äußerst ungünstig sind. Von dem gesamten Reaktorvolumen, welches sich aus den Volumina der Innenräume der Kapillaren (Vc), deren Wandvolumina (Vw) (abzüglich der Gerüstvolumina) und (' des verbleibenden Volumens (Vo) zwischen den Kapillaren zusammensetzt, steht nur das Innere der Kapillarmembranen für das produzierende System zur Verfügung. Das Kapillarwand- und verbleibende Volumen zwischen den Einzelkapillaren ist mit dem zu-/abführenden System erfüllt. Unter diesen Voraussetzungen werden selbst bei Reaktoren mit extrem vielen Kapillaren nur ungünstige Phasenverhältnisse erzielt, wie sich aus der

3

nachstehenden Tabelle ergibt, in der das Volumenverhältnis des produzierenden Systems zum Versorgungssystem einiger in die Untersuchung einbezogener Bioreaktoren zusammengefaßt dargestellt ist. Dabei haben alle Reaktoren einen Innendurchmesser von 10 mm und eine Länge von 50 mm. Die verwendeten Kapillarmembranen haben einen Innendurchmesser von 0,8 mm und einen Außendurchmesser von 1,3 mm.

| Kapillaren | Vc | Vw | Vo | Vprod./Vvers. |
|---|---|---|---|---|
| (Stk.) | | µl | | |
| 5 | 70 | 330 | 3525 | 0,018 |
| 10 | 140 | 660 | 3125 | 0,037 |
| 15 | 210 | 990 | 2725 | 0,056 |
| 20 | 280 | 1320 | 2325 | 0,077 |
| 32 | 448 | 1664 | 1813 | 0,129 |

Es zeigt sich, daß unter den der Tabelle zugrundeliegenden Voraussetzungen der für das produzierende System zur Verfügung stehende Raum nur etwa 1/8 des vom Versorgungssystem eingenommenen Volumens beträgt.

Der Erfindung liegt die Aufgabe zugrunde, ein Verfahren der eingangs genannten Art zu schaffen, welches die Entstehung einer den Verfahrensablauf beeinträchtigenden Sekundärfilterschicht mit allen sich daraus ergebenden negativen Konsequenzen wirksam verhindert, ohne daß die durch die Membran isolierten Biomakromoleküle, Zellen oder sonstigen biologisch aktiven Partikel schädlichen Scherkräften ausgesetzt werden.

Vorstehende Aufgabe wird erfindungsgemäß dadurch gelöst, daß die Bewegungsrichtung der die Membran durchdringenden Stoffe durch Druckänderungen in zeitlichen Abständen vorübergehend umgekehrt wird.

Durch die vorgeschlagenen Druckänderungen kommt es in und auf der Membran zu völlig anders gearteten Flußprofilen als bei den bisher bekannten Bioreaktoren. Dies gilt grundsätzlich für Reaktoren mit Membranen jeder Form und Gestalt. Abhängig von der Dauer und Intensität der Druckphasen werden bedeutend raschere Austauschgeschwindigkeiten erzielt. Bei einem Diffusionsreaktor können z. B. die Halbwertszeiten von mehr als einem Tag auf wenige Minuten verkürzt werden.

Die vorgeschlagenen Druckänderungen, welche vorzugsweise als Druckmodulation mit regelmäßiger Frequenz und Amplitude durchgeführt werden, können in jedem Anwendungsfall so gewählt und optimiert werden, daß es überhaupt nicht mehr zur Ausbildung einer Sekundärfilter- bzw. Deckschicht kommt. Es kann kontinuierlich unter den optimalen Bedingungen gearbeitet werden, die in den herkömmlichen Membran-Reaktoren jeweils nur kurzzeitig als Anfangsbedingungen zur Verfügung standen. Der Filtratfluß ist zeitunabhängig. Ohne Deckschicht kommt es auch nicht mehr zur Ausdünnung des produzierenden Systems, und es können über den gesamten Zeitraum hinweg bei optimalem Wirkungsgrad größtmögliche Umsetzungsraten erzielt werden. Die Austauschgleichgewichte sind identisch mit denen der klassischen Membran-Reaktoren. Die Ver- und Entsorgung des produzierenden Systems ist auf Dauer problemlos gewährleistet. Durch die Druckmodulation ergeben sich Strömungsverhältnisse, die praktisch scherkraftfrei sind und somit in keiner Weise einen Aktivitätsverlust biologischer Systeme hervorrufen.

Es hat sich bei dem neuen Verfahren als zweckmäßig erwiesen, die Druckänderungen ausreichend stark und lang zu bemessen, um mindestens ein dem Hohlraumvolumen der Membran entsprechendes Flüssigkeitsvolumen in der jeweiligen Druckrichtung durch die Membran zu fördern.

Als Vorrichtung zur Durchführung des oben angegebenen Verfahrens wird ein Bioreaktor vorgeschlagen, bei welchem an den Reaktionsraum und den Versorgungsraum je ein Druckerzeuger angeschlossen sind, die abwechselnd betätigbar sind. Dabei wird insbesondere ein Bioreaktor bevorzugt, dessen Membran aus einer Vielzahl von Kapillarmembranen mit einer äußeren Filtrationsschicht auf einer inneren, größerporigen Tragschicht besteht und dessen Reaktionsraum sich außerhalb der Kapillarmembranen befindet.

Die Erfindung wird nachstehend anhand der Zeichnung näher erläutert. Es zeigen:

Fig. 1 einen vereinfachten senkrechten Längsschnitt durch einen nach dem erfindungsgemäßen Verfahren zu betreibenden Bioreaktor mit Kapillarmembranen;

Fig. 2 einen Querschnitt durch einen Bioreaktor mit Kapillarmembranen;

Fig. 3 ein Diagramm zur Veranschaulichung des zeitlichen Verlaufs der Durchflußrate bei einem herkömmlichen Bioreaktor;

Fig. 4 ein Diagramm zur Veranschaulichung der sich im Zeitablauf ändernden Strömungsrichtung bei einem nach dem erfindungsgemäßen Verfahren betriebenen Bioreaktor;

Fig. 5 ein Schaubild entsprechend Fig. 4, welches die Strömungsverhältnisse bei abgewandelter Druckmodulation darstellt;

Fig. 6 ein Schaubild, welches Konzentrationsänderungen nach Beginn einer Druckmodulation, im Bioreaktor gemäß Fig. 4 oder 5 zeigt;

Fig. 7 einen schematischen Längsschnitt durch einen Bioreaktor, bei welchem sich das produzierende System auf der Außenseite von Kapillarmembranen befindet, zusammen mit verschiedenen an den Reaktionsraum und den Versorgungsraum anzuschließenden Druckerzeugern.

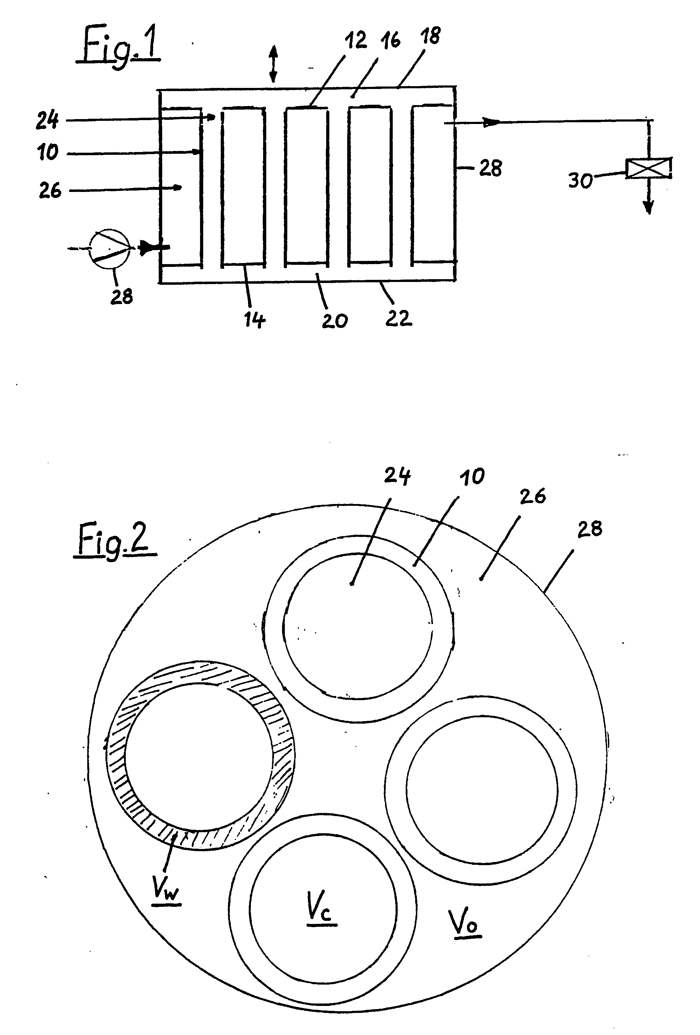

Der in Fig. 1 dargestellte Bioreaktor enthält eine Vielzahl von Kapillarmembranen 10, die am oberen und unteren Ende in Abschlußwände 12 und 14 dicht eingelassen, z. B. eingegossen, sind, wobei jedoch die oberen Öffnungen aller Kapillarmembranen 10 über einen oberen Hohlraum 16 zwischen der Abschlußwand 12 und einer den Bioreaktor oben abschließenden undurchlässigen Membran 18 miteinander in Verbindung stehen. In entsprechender Weise sind auch die unteren Öffnungen der Kapillarmembranen 10 über einen gemeinsamen Hohlraum 20 zwischen der Abschlußwand 14 und einer unteren Endwand 22 miteinander verbunden.

Ein "produzierendes System", z. B. eine Zellkultur, befindet sich im Innenraum 24 der Kapillarmembranen 10. Dieser Raum sei als Reaktionsraum bezeichnet. Der die Kapillarmembranen 10 umgebende Zwischenraum 26, welcher von der Mantelwand 28 des Reaktors nach außen begrenzt wird, enthält eine Nährlösung und sei deshalb als Versorgungsraum bezeichnet.

Während des Betriebs des Bioreaktors wird dem Versorgungsraum 26 über eine Dosierpumpe 28 Nährlösung zugeführt, welche die Kapillarmembranen 10 umspült. Der Abfluß der Nähr lösung aus dem Versorgungsraum 26 erfolgt über ein Rückdruckventil 30. Wird dieses vorübergehend geschossen, so baut sich in dem Bioreaktor ein Druck auf, der einen Fluß von Nährlösung durch die Kapillarmembranen 10 in deren Innenraum 24, d. h. zu dem produzierenden System, hervorruft. Das durch die Kapillarwände dringende Volumen kann wegen der flexiblen Ausgleichsmembran 18 im oberen Hohlraum 16 zusätzlich aufgenommen werden, wobei sich die Membran 18 nach oben aufwölbt. Nach einer bestimmten Zeitdauer wird dann das Rückdruckventil 30 wieder geöffnet. Indem die Ausgleichsmembran 18 nunmehr von außen z. B. pneumatisch oder mechanisch mit Druck beaufschlagt und in Richtung des eingezeichneten Doppelpfeils nach unten zurückgedrängt wird, kann das in die Kapillarmembranen 10 eingedrungene Flüssigkeitsvolumen wieder radial nach außen durch die Membranwände hindurch in den Versorgungsraum 26 zurücktransportiert werden. Hierbei gelangen neben nicht umgesetzten "Nährstoffen" auch die vom produzierenden System erzeugten Produkte, z. B. Polypeptide, in den Versorgungsraum 26. Das produzierende System wird also entsorgt.

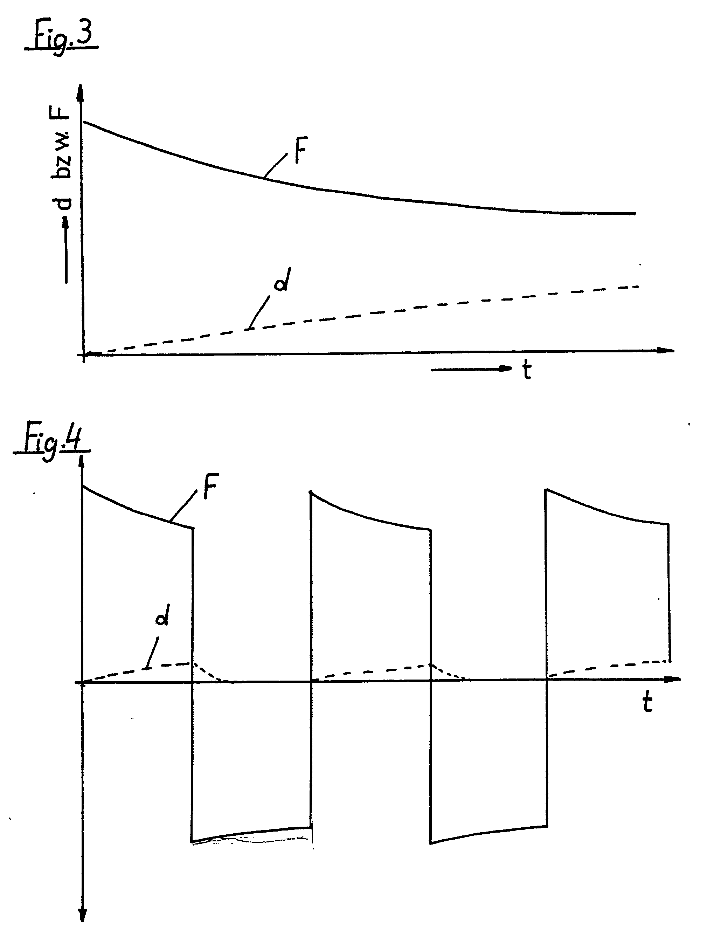

In der zuletzt genannten Strömungsphase kommt es auch zur Ausbildung des oben erwähnten Konzentrationsgradienten auf der Innenseite der Kapillarmembranen 10. Würde man, wie bisher, kontinuierlich und ohne Abwechslung den Druck im Innenraum der Kapillarmembranen etwas größer wählen als in dem der Versorgung und Entsorgung dienenden Zwischenraum 26, so ergäben sich für den Fluß F durch die Kapillarwände und für die Dicke d der sich an der Innenseite der Kapillarwände anlagernden Deckschicht im Laufe der Zeit die in Fig. 3 ausgezogen bzw. gestrichelt dargestellten Kurven. Dies bedeutet, daß die Deckschicht zunehmend dicker wird und den Durchfluß durch die Membranwand zunehmend drosselt.

Wird dagegen bei dem Bioreaktor nach Fig. 1 das Rückdruckventil 30 abwechselnd jeweils für eine bestimmte Zeitdauer geschlossen und geöffnet, um die erfindungsgemäß vorgeschlagene Strömungsumkehr zu erhalten, so ergeben sich für Fluß und Deckschicht die in Fig. 4 dargestellten Kurven. Wie ersichtlich, baut sich hier nur jeweils während derjenigen Phase, in welcher der Druck im Reaktionsraum 24 den Druck im Versorgungsraum 26 überwiegt, eine Sekundärfilterschicht auf, deren Dicke jedoch durch die Bemessung der Zeitdauer dieser Phase begrenzt wird. In der anschließenden Phase ergibt sich bei umgekehrter Druckrichtung eine Gegenströmung vom Versorgungsraum 26 radial durch die Membranwände in den Innenraum 24 der Kapillarmembranen 10, wobei die an der Innenseite der Membranwände angelagerte Deckschicht wieder vollständig beseitigt wird. Insgesamt läßt sich feststellen, daß der Bioreaktor bei der hier vorgeschlagenen Betriebsweise mit Druckmodulation zur alternierenden Strömungsumkehr kontinuierlich und auf Dauer unter den günstigen Anfangsbedingungen arbeitet, welche bei einem herkömmlichen Bioreaktor nur zu Beginn seines Betriebs vorhanden sind. Demnach wiederholt sich in den oberhalb der Zeitachse aufgetragenen Kurvenabschnitten F gemäß Fig. 4 der optimal hohe Anfangsabschnitt der Druckflußkurve F gemäß Fig. 3.

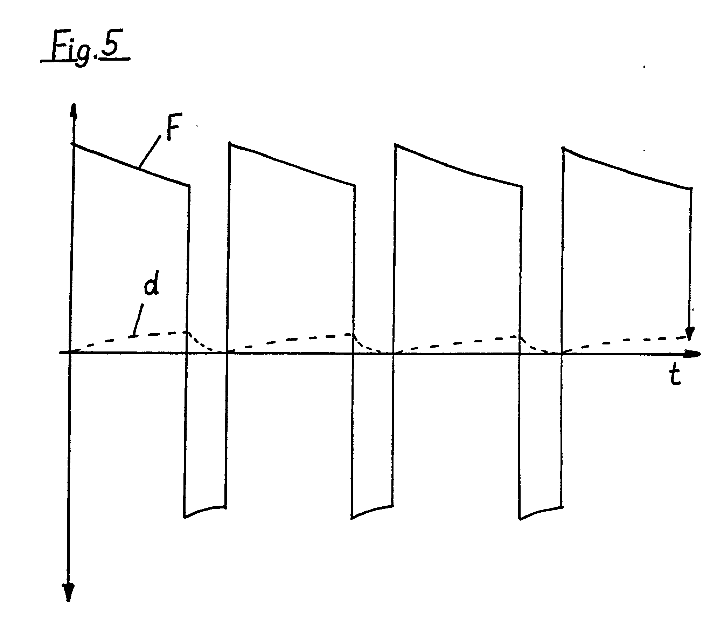

Aus Fig. 4 geht weiterhin hervor, daß die Druckschwankungen nach der einen und nach der anderen Seite weder gleichlang noch gleichgroß zu sein brauchen, um das angestrebte Ziel der immer wiederkehrenden Beseitigung schon der anfänglichen Anlagerungen der Deckschicht zu erreichen. Deshalb läßt sich gemäß Fig. 5 in jedem Einzelfall der Kurvenverlauf der Druck- bzw. Flußänderungen so optimieren, daß die Phase der Gegenströmung nicht länger dauert als notwendig, um die während der vorangegangenen Phase gebildete Deckschicht wieder zu beseitigen. Selbstverständlich müssen hierbei die Betriebsparameter so eingestellt werden, daß das produzierende System auf Dauer ausreichend mit Flüssigkeit und "Nährstoffen" versorgt wird. Dies kann über einen in Fig. 1 nicht gezeigten, ventilgesteuerten Zulauf für Nährlösung zum Raum 20 erfolgen.

Im Gegensatz zu durchströmten Reaktoren kommt es bei dem neuen Reaktortyp, der mit einer Druckmodulation nach Fig. 4, Fig. 5 oder einer ähnlichen Kurve betrieben wird, zu keinem echten Durchfluß mehr. Dadurch werden die bisher aufgetretenen Probleme des Strofftransports von der Membranoberfläche bzw. aus der Deckschicht in die Rohlösung bzw. Biomasse etc. überwunden. Der Durchtritt der Nährlösung usw. vom Außenraum ins Innere der Kapillarmembranen bewirkt einen Fluß, ähnlich dem in aktiv durchströmten Systemen, der die von der Membran zurückgehaltenen Makromoleküle abtransportiert und mit dem produzierenden System wieder vermischt. Dieser Vermischungsvorgang findet jedoch unter besonders milden Bedingungen statt, so daß eine Schädigung des produzierenden (Bio)Systems praktisch ausgeschlossen ist.

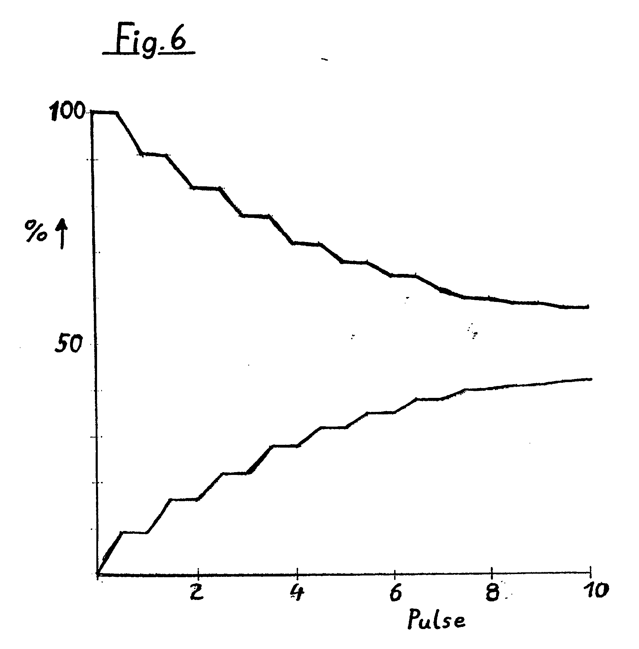

Die vorteilhafte Wirkung der Erfindung läßt sich auch anhand von Fig. 6 demonstrieren. Es wird davon ausgegangen, daß vor Beginn der Druckmodulation die Konzentration bestimmter, vom produzierenden System erzeugter Stoffe im Reaktionsraum 100 % betrage. Dem/gegenüber sei auf der anderen Seite der Membran, im Versorgungsraum die Konzentration dieser Stoffe bei Null. Wenn jetzt die Pulsation des Drukkes einsetzt, sieht man anhand von Fig. 6, wie die mit der oberen Kurve dargestellte Konzentration der betreffenden Stoffe im Reaktionsraum mit jedem Puls (bestehend aus Phase und Gegenphase) des Drucks Schritt für Schritt abfällt und sich dem Wert von 50 % annähert. Umgekehrt steigt die untere Kurve, welche die Konzentration im Versorgungsraum darstellt, gegenläufig an.

Das Volumen, welches bei jedem Druckpuls in einer Richtung durch die Membranwand gefördert wird, sollte mindestens in der Größenordnung des Totvolumens (Hohlraumvolumens) der Membranwand sein. Dadurch wird die Diffusionsbarriere überwunden, und es werden Austauschgleichgewichte in einer Größenordnung gefunden, wie sie an unendlich dünnen Membranen bzw. an Flüssig-Flüssig-Grenzflächen auftreten. Durch den geringen Fluß durch die Membranen kommt es auf der Membranoberfläche zu einem Transportmechanismus, der eine milde Durchmischung der Rohlösung bzw. Biomasse bewirkt und die Effizienz des Reaktors weiter steigert. Typische Mindestmodulationsvolumina liegen, abhängig von der Wandstärke des Ultamembranfilters, in der Größenordnung von 5 - 100 $\mu$lcm$^{-2}$. Für Folienmembranen werden kleinere Werte gefunden.

Bei durchmischten Systemen mit einer überströmten Membran sollte das bei jedem Druckpuls in einer Richtung durch die Membran geförderte Modulationsvolumen mindestens so groß sein, daß der größte Teil der zurückgehaltenen Moleküle in den gerührten bzw. durchmischten Bereich über der Membran gelangt und so der Abtransport "aktiv" gewährleistet ist. Abhängig von der Intensität der Durchmischung, werden Größenordnungen im Bereich von wenigen $\mu$lcm$^{-2}$ bis einige hundert $\mu$lcm$^{-2}$ erforderlich, in besonderen Fällen auch noch höhere Werte.

Da durch die Flußmodulation Flüsse entgegengesetzter Richtungen entstehen, ist es leicht möglich, dieses Phänomen durch geeignete Flußführung als Vermischungs- und Optimierungsmechanismus einzusetzen. Die Modulationsvolumina werden dann selbst zum Operationsparameter, was zur Folge hat, daß auch bedeutend größere Volumina, als oben angegeben, sinnvoll werden können.

Wie aus Fig. 2, wo ein Querschnitt durch einen Bioreaktor der Bauart nach Fig. 1 dargestellt ist, hervorgeht, ergeben sich normalerweise dann ungünstige Phasen- bzw. Volumenver hältnisse, wenn sich das produzierende System im Innenraum 24 der Kapillarmembranen 10 und das zu/abführende System im Raum außerhalb der Kapillaren befindet. Es sind oben bereits in einer Tabelle beispielhaft einige Volumenverhältnisse herkömmlicher Bioreaktoren angegeben worden.

Günstigere Phasenverhältnisse können erfindungsgemäß dadurch erzielt werden, daß man die Räume für die beiden Phasen vertauscht. Hierzu werden Ultramembrankapillaren benötigt, welche die eigentliche Filterschicht auf der Außenseite tragen. Derartige Membranen sind bekannt und werden in sog. Hohlfasermodulen z. B. zur Entsalzung von Brackwasser eingesetzt. Die bekannten Membranfasern haben zwar den geforderten asymmetrischen Aufbau mit der Trennschicht auf der Außenseite, ihre Innendurchmesser betragen aber im allgemeinen nur 80 - 100 um. In der praktizierten Anwendung wird eine Rohlösung von außen in ein Mantelrohr gepumpt und auf der anderen Seite als Konzentrat abgepumpt. Das Filtrat durchdringt die Fasern von außen nach innen. Nachteilig ist, daß es auf der Membranoberfläche zu Niederschlägen kommt, die kaum entfernt werden können. Deshalb wird diese Trenntechnik im Bereich von makromolekularen und kolloidalen Lösungen, also auch der Biotechnologie, bisher nicht eingesetzt.

Die Erfindung bietet nunmehr die Möglichkeit, Bioreaktoren zu bauen, bei denen sich die Nährlösung in den Kapillaren und das produzierende System außerhalb derselben befindet. Notwendige Voraussetzung hierfür ist die vorgeschlagene Druckmodulation, denn nur sie bietet die Gewähr dafür, daß sich außen auf den Kapillarmembranen keine zunehmend dicker werdende Deckschicht bildet. Außerdem hat es sich als zweckmäßig erwiesen, die Durchmesser etwas größer zu wählen als bei den für die Wasserentsalzung verwendeten Hohlfasermembranen, um zu hohe Fließdrücke zu vermeiden. In welchem

Maße nunmehr die Möglichkeit besteht, ein geeignetes Volumenverhältnis von produzierendem und Versorgungssystem zu erhalten, zeigt die nachstehende Tabelle, der ein Bioreaktor mit einem Innendurchmesser von 10 mm und einer Länge von 50 mm zugrunde gelegt ist. Angegeben sind unterschiedliche Stückzahlen von Kapillaren, deren Innendurchmesser (ID), Außendurchmesser (OD) sowie die aus den genannten Maßen errechneten Volumina und Volumenverhältnisse.

| Kapillaren | ID | OD | $V_c$ | $V_w$ | $V_o$ | $V_{prod.}/V_{vers.}$ |
|---|---|---|---|---|---|---|
| (Stk) | (mm) | (mm) | ($\mu$l) | ($\mu$l) | ($\mu$l) | |
| 5 | 0,1 | 0,4 | 2,5 | 30 | 3895 | 120 |
| 10 | 0,1 | 0,4 | 5 | 60 | 3860 | 59 |
| 25 | 0,1 | 0,4 | 12,5 | 150 | 3762 | 23 |
| 32 | 0,1 | 0,4 | 16 | 192 | 3717 | 18 |
| 40 | 0,1 | 0,4 | 20 | 240 | 3665 | 14 |
| 5 | 0,8 | 1,4 | 125 | 260 | 3540 | 12 |
| 10 | 0,8 | 1,4 | 250 | 520 | 3155 | 4 |
| 20 | 0,8 | 1,4 | 500 | 1040 | 2385 | 1,5 |
| 26 | 0,8 | 1,4 | 650 | 1352 | 1923 | 0,85 |

Fig. 7 zeigt einen solchen Bioreaktor, bei dem sich das produzierende System auf der Außenseite von Kapillarmembranen 11 befindet, die Zufuhr von Nährlösung durch den Innenraum 24 der Kapillarmembranen 11 erfolgt und durch Druckmodulation dafür gesorgt wird, daß sich auf der Außenseite der Kapillarmembranen keine störende Deckschicht bildet. Im Gegensatz zu der Ausführung nach Fig. 1 haben die Kapillarmembranen 11 bei dem Ausführungsbeispiel nach Fig. 7 die eigentliche Filterschicht außen und die großporige Tragschicht innen. Die Nährlösung wird durch die Dosierpumpe 28 aus einem Vorratsbehälter 29 in den oberen Hohlraum 16 gepumpt, mit welchem ebenso wie bei Fig. 1 sämtliche Kapillarmembranen in Verbindung stehen. Die Anschlußstelle ist mit X bezeichnet. Auf der anderen Seite befindet sich bei Y ein Abfluß aus dem unteren Hohlraum 20. Die Ablaufleitung führt über ein Ventil 31 zu einer Extraktionseinrichtung, wo die vom produzierenden System erzeugten Stoffe aus der Lösung bzw. Suspension gewonnen werden.

Abgesehen von den vorstehend erwähnten Besonderheiten der Kapillarmembranen 11 und der Anschlüsse kann der Reaktor nach Fig. 7 denselben mechanischen Aufbau haben wir der Reaktor nach Fig. 1. Ergänzend sei noch auf einige alternativ in Frage kommenden Einrichtungen zur Druckmodulation eingegangen, die in Fig. 7 schematisch dargestellt sind. Es sei zunächst auf den bei 32 gezeigten Druckerzeuger eingegangen.

Der Druckerzeuger 32 ist mit seinem einen Ausgang 34 an den Auslaß Y oder alternativ an den Einlaß X oder beide parallel angeschlossen. Der andere Ausgang 36 des Druckerzeugers 32 ist über einen Anschluß Z an den die Kapillarmembranen 11 umgebenden Zwischenraum 26 im Reaktor angeschlossen. Die Funktion des Druckerzeugers 32 besteht darin, daß er abwechselnd über seinen Ausgang 34 den Innenraum 24 der Kapillarmembranen 11 und über seinen Ausgang 36 die Außenseite der Kapillarmembranen 11 mit Druck beaufschlagt. Dies geschieht mittels zweier Zylinder 38 und 40, deren Kolben einen auf sie wirkenden Gasdruck, angedeutet als $N_2$, auf die jeweils angeschlossenen Flüssigkeitsvolumina übertragen. Jedem Zylinder 38 bzw. 40 ist ein umsteuerbares Ventil 42 bzw. 44 vorgeschaltet, und diese Ventile sind so gesteuert, daß sie den dauernd anliegenden Gasdruck abwechselnd mit der vorgesehenen Modulationsfrequenz auf jeweils einen der Zylinder 38 und 40 geben, während der jeweils andere Zylinder vom Gasdruck entlastet wird. Es versteht sich, daß bei Druckbeaufschlagung eines der beiden Flüssigkeitsvolumina vorhandene Flüssigkeits-Anschlußleitungen abzusperren sind, damit sich auf der jeweiligen Seite des Reaktors der vorgesehene Druck aufbauen kann.

Neben dem Druckerzeuger 32 ist als weitere Alternative ein Druckerzeuger 46 gezeigt, welcher ebenso wie der Druckerzeuger 32 an den Bioreaktor anzuschließen ist und sich nur dadurch unterscheidet, daß statt der Zylinder 38, 40 mit darin verschieblichen Kolben flexible, undurchlässige Membranen 48 bzw. 50 vorhanden sind, welche den Gasdruck auf die abwechselnd mit Druck zu beaufschlagenden Flüssigkeitsvolumina übertragen. Selbstverständlich können die Membranen 48, 50 und die Kolben in den Zylindern 38, 40 auch auf jede andere geeignete Weise, z. B. elektromechanisch oder hydraulisch hin und her bewegt werden, um die vorgesehene Druckmodulation zu erzeugen.

**Ansprüche**

1. Verfahren zur Beschleunigung des Stoffaustauschs eines kontinuierlichen Bioreaktors, bei welchem innerhalb einer Flüssigkeit ein Reaktionsraum (24 bzw. 26) durch eine semipermeable Membran (10 bzw. 11) von einem Versorgungsraum (26 bzw. 24) betrennt ist, **dadurch gekennzeichnet,** daß die Bewegungsrichtung der die Membran (10 bzw. 11) durchdringenden Stoffe durch Druckänderungen in zeitlichen Abständen vorübergehend umgekehrt wird.

2. Verfahren nach Anspruch 1, **dadurch gekennzeichnet,** daß die Druckänderungen als Druckmodulation mit regelmäßiger Frequenz und Amplitude durchgeführt werden.

3. Verfahren nach Anspruch 1, **dadurch gekennzeichnet,** daß die entgegengesetzten Phasen der Druckwellen eine unterschiedliche Dauer und/oder Amplitude haben.

4. Verfahren nach Anspruch 1, **dadurch gekennzeichnet,** daß die Druckänderungen in Abhängigkeit von der über die Membran (10; 11) gemessenen Druckdifferenz oder der Konzentration von Stoffen unmittelbar neben dieser gesteuert werden.

5. Verfahren nach einem der Ansprüche 1 bis 4, **dadurch gekennzeichnet,** daß während der Druckänderungen die Flüssigkeit im Reaktionsraum (24 bzw. 26) in Strömungsbewegung parallel zur Oberfläche der Membran (10; 11) versetzt wird.

6. Verfahren nach einem der vorhergehenden Ansprüche, **dadurch gekennzeichnet,** daß die Druckänderungen ausreichend stark und lang bemessen sind, um mindestens ein dem Hohlraumvolumen der Membran (10; 11) entsprechendes Flüssigkeitsvolumen in der jeweiligen Druckrichtung durch die Membran (10; 11) zu fördern.

7. Bioreaktor zur Durchführung des Verfahrens nach einem der Ansprüche 1 bis 6, **dadurch gekennzeichnet,** daß an den Reaktionsraum (24 bzw. 26) und den Versorgungsraum (26 bzw. 24) je ein Druckerzeuger (38, 40; 48, 50) angeschlossen sind, die abwechselnd betätigbar sind.

8. Bioreaktor nach Anspruch 7, **dadurch gekennzeichnet,** daß die Membran (11) aus einer Vielzahl von Kapillarmembranen mit einer äußeren Filtrationsschicht auf einer inneren, größerporigen Tragschicht besteht und sich der Reaktionsraum (26) außerhalb der Kapillarmembranen (11) befindet.

9. Bioreaktor nach Anspruch 7 oder 8, **dadurch gekennzeichnet,** daß an wenigstens einem Ende der Kapillarmembranen (10; 11) ein deren Öffnungen verbindender Hohlraum (16) durch eine undurchlässige, flexible Membran (18) abgeschlossen ist, deren Außenseite mit veränderbarem Druck beaufschlagbar ist.

Fig.1

Fig.2

Fig.3

Fig.4

Fig.5

Fig.6

EP 0 398 083 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WO-A-8 602 378 (ENDOTRONICS INC.) * Anspr.; Figuren 2,3; Seite 7, Zeilen 9-20; Seite 9, Zeile 17 - Seite 11, Zeile 15 * --- | 1-9 | C 12 M 1/12 C 12 M 3/00 |
| X | EP-A-0 073 079 (Dr. MULLER AG) * Anspr.; Fig. Beisp. * --- | 1,2,6 | |
| X | EP-A-0 007 133 (CHEMAP AG) * Anspr.; Figur 3; Seite 7, Zeilen 1-8 * --- | 1,2,6 | |
| X | DE-A-3 605 065 (DIESSEL GmbH) * Ganzes Dokument * ----- | 1-9 | |

**RECHERCHIERTE SACHGEBIETE (Int. Cl.5)**

C 12 M

Der vorliegende Recherchenbericht wurde für alle Patentansprüche erstellt

| Recherchenort | Abschlußdatum der Recherche | Prüfer |
|---|---|---|
| DEN HAAG | 03-09-1990 | COUCKE A.O.M. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument